# EUROPEAN PATENT SPECIFICATION

(11) **EP 3 072 468 B1**
(45) Date of publication and mention of the grant of the patent: **18.09.2019**
(21) Application number: 16275043.4
(22) Date of filing: 09.03.2016
(51) Int. Cl.: A61B 18/14

(54) **MEDICAL ABLATION SYSTEM WITH REDUCED STRAY HEATING**
MEDIZINISCHES ABLATIONSSYSTEM MIT REDUZIERTER STREUHEIZUNG
SYSTÈME D'ABLATION MÉDICAL AVEC CHAUFFAGE PARASITE RÉDUIT

(30) Priority: 26.03.2015 GB 201505150
(43) Date of publication of application: 28.09.2016
(73) Proprietor: Cook Medical Technologies LLC, Bloomington, IN 47404 (US)
(72) Inventor: PAAMAND, Rune T., 2700 Broenshoej (DK); TORP, Allan, 4632 Bjaeverskov (DK); BUTZBACKER, Raimo Urban, 4690 Haslev (DK); PEDERSEN, Per Elgaard, 4690 Haslev (DK)
(74) Representative: Williams Powell

(56) References cited:
- US-A- 6 022 346
- US-A1- 2012 220 999
- US-A1- 2012 265 200
- US-A1- 2014 214 140
- US-B1- 6 358 273

## Description

### Technical Field

The present invention relates to a medical ablation system, or apparatus for effecting ablation within a patient, for instance for causing blood clotting within a vessel, for treating an organ or a medical condition, or the like.

### Background Art

There are numerous medical conditions for which it is desired or necessary to close a body vessel, including for instance in the treatment of aneurysms, arteriovenous malformations, arteriovenous fistulas, for starving organs of oxygen and nutrients for instance in the treatment or containment of cancerous growths, and so on. Ablation can be also be used to destroy diseased tissue, including cancers and the like.

Several techniques are known and in use for closing or occluding body vessels. Traditionally, vessels have been closed by means of external ligation, which generally must be carried out by an open surgical procedure, with its associated risks, inconvenience and long patient recovery times. Other more recent methods aim to use an endoluminal procedure to insert into the vessel or organ one or more occlusion devices, such as embolization coils, particles or the like, able to obstruct the flow of blood in the vessel.

Attempts have also been made to constrict a vessel by endoluminal ablation, causing contraction and fusing of the vessel and/or coagulation of blood to form a blood clot in the vessel. A technique which has been considered suitable is RF ablation, in which an electrical terminal is fed endoluminally into the vessel and an electrical pulse at RF frequencies applied through the electrical terminal. The conductivity of blood and/or the vessel tissues causes localised heating, which can be used to cause damage to the tissue (intima) of the vessel wall, resulting in vessel contraction. In other devices RF ablation heats the surrounding blood, causing this to coagulate around the electrical terminal, forming a blood clot which blocks the vessel. Ablation systems for these purposes may be monopolar, having one electrode located in the vessel to be treated and another electrode, in the form of a conductive pad, placed against the patient's skin at a location closest to the vessel to be treated. Other systems are bipolar, having both conductive electrodes located at the distal end of an elongate introducer element. A bipolar system does not require an external terminal.

Ablation systems are not necessarily limited to use of RF energy as they can rely on resistive heating, optical heating and so on.

In order to effect ablation, it is generally necessary to heat the blood plasma or tissue to an elevated temperature for a period of time. Temperatures in excess of 60 to 70 degrees Centigrade are typically required.

A principal problem with endoluminal ablation systems lies with managing the heat generated during the process and resultant undesired damage to adjacent tissues, nerves or the patient's organs, particularly those at or proximate the internally positioned electrode or electrodes. The risk of collateral damage of this type has limited the use and application of endoluminal ablation systems.

The required energy needed for successful endoluminal ablation is increased due to the heat sink effect of the blood flow. Furthermore an RF electrode has a maximum power capacity proportional to its size, above which the electrode will cause charring and become less conductive, which in turn limits its ability to deliver energy. Examples of prior art devices and methods can, for instance, be found in US-2010/0331666, US-2013/0281851, US-2014/0018697, US-2014/0012132, US-2014/0018788, US-2012/0172867, US-2012/0283562, US-2010/0185087, US-2012/265200, US-6022346, US6358273, US-2014/214140.

US-2012/265200 describes a device for ablation therapy with a pre-heating element and an ablation element.

US-6022346 describes a system for ablation therapy with a pre-heating element, an ablation element and a controller setting a pre-heating temperature and an ablation temperature.

US-2012/220999 describes an electrical ablation device providing pre-heating to a tissue at a temperature above 40°C by electrodes.

### Summary of the Invention

The present invention seeks to provide an improved system for effecting medical ablation.

According to the invention there is provided an ablation system as in claim 1. Preferred embodiments of the invention are illustrated in the dependent claims.

The system provides for pre-heating of a vessel or tissue to be ablated, at a first lower temperature, which typically does not cause blood coagulation, or organ or tissue damage. Such preheating reduces the amount of energy at ablation power which is subsequently required to effect the ablation step, resulting in the avoidance of exceeding the power capacity of the electrode and a reduction in collateral tissue damage during the treatment.

In some embodiments, the electrical ablation element is at least one electrical ablation terminal.

In some embodiments, the electrical ablation element includes a resistive heating device. For example, the electrical ablation element may include a resistive element which is electrically insulated, for example by means of a polymer coating such as PTFE.

Preferably, the ablation assembly is for deployment in a blood vessel and the heating device is longitudinally spaced from the electrical ablation element so as to be able to provide longitudinally spaced heating. This can allow blood to be preheated upstream of the electrical ablation element on its way to the ablation element so that the blood arriving at the ablation element is preheated and less time and energy is required to heat the blood to ablation temperatures. This is advantageous over heating the blood in the location of the ablation element as, in the latter instance, preheated blood may flow away from the ablation element before ablation occurs.

Preferably, the control unit is configured to actuate the heating device to heat to a temperature above body temperature, for instance to a temperature below around 60 degrees Centigrade, in one example to around 50 degrees Centigrade. The control unit is also preferably configured to actuate the electrical ablation element to heat to a temperature above blood coagulation temperature, for example to actuate the electrical ablation element, for example to heat, to a temperature above about 60 degrees Centigrade, for instance to about or around 65 or 70 degrees centigrade. The desired ablation temperature will be dependent upon the ablation target, for instance whether blood is to be coagulated, tissue is to be ablated, diseased cells are to be destroyed, and so on. The skilled person will be able to determine the required temperatures, either from common general knowledge or by routine experimentation.

Preferably, the control unit is operable to actuate the heating device before the electrical ablation element. The first temperature advantageously provides preheating prior to application of the second temperature or generation of heating at the second temperature. The heating device may continue to be operated during the period of operation of the electrical ablation element, although in other embodiments the heating device may be disabled once the ablation element is activated.

The heating device may be a resistive heating device. In an embodiment, the heating device is electrically separate from the electrical ablation element. In some embodiments, the heating device may include an electrical connector in common with the electrical ablation element.

Advantageously, the heating device is a closed loop heating element.

In a preferred embodiment, the at least one electrical ablation terminal is an RF ablation terminal. The elongate ablation assembly may include one or a single electrical ablation terminal, forming a first electrode of a monopolar ablation apparatus. In other embodiments, the elongate ablation assembly may include at least two electrical ablation terminals, forming the electrodes of a bipolar or multipolar ablation apparatus.

In practical embodiments, the elongate ablation assembly may include a temperature sensor disposed to measure temperature at least at the heating device.

The assembly may include the optional features indicated above and elsewhere in this specification.

In some embodiments, the assembly includes a distal end and a proximal end, the electrical ablation element being disposed at the distal end of the device, the heating device being disposed at or proximate the distal end.

Also described herein is a method of effecting ablation in a body vessel including the steps of:
deploying in a patient's vessel an ablation assembly including a heating device and an electrical ablation element;
actuating the heating device to provide heating to a first temperature; and
actuating the electrical ablation element to provide heating at a second temperature, the first temperature being at below an ablation temperature and the second temperature being at or above an ablation temperature;
wherein the first temperature provides vessel preheating.

Also described herein is a method of effecting ablation in a body vessel, including the steps of: deploying in a patient's vessel an ablation assembly having a proximal end and a distal end, and provided at or adjacent its distal end with a heating device and an electrical ablation element; actuating the heating device to provide heating to a first temperature; and actuating the electrical ablation element to provide heating at a second temperature, the first temperature being at below an ablation temperature and the second temperature being at or above an ablation temperature; wherein the first temperature provides vessel preheating.

Preferably, the first temperature is around 50 degrees Centigrade and the second temperature is around 65 to 70 degrees Centigrade, preferably around 70 degrees Centigrade.

Preferably, the patient's vessel is a blood vessel and the heating device is longitudinally spaced from the electrical ablation element.

The method preferably includes actuating the heating device and the electrical ablation element so as to provide longitudinally spaced heating. This can allow blood to be preheated upstream of the electrical ablation element on its way to the ablation element so that the blood arriving at the ablation element is preheated and less time and energy is required to heat the blood to ablation temperatures.

The method preferably includes positioning the heating device upstream from the electrical ablation element with respect to blood flow.

The method preferably includes the step of heating at the first temperature before heating at the second temperature.

Other features and advantages are described below in connection with the preferred embodiments.

### Brief Description of the Drawings

Embodiments of the present invention are described below, by way of example only, with reference to the accompanying drawings, in which:
Figure 1 is a diagram of a first embodiment of dual temperature ablation device;
Figure 2 is a schematic view of the device of Figure 2;
Figure 3 is a diagram of another embodiment of dual temperature ablation device;
Figure 4 is a block diagram of the principal components of an embodiment of control unit for the system taught herein;
Figure 5 is a sketch of a device as taught herein during its deployment in a patient's vessel;
Figure 6 is a flow diagram showing the operation of the control unit of Figure 4;
Figure 7 is a view of an electrical ablation element for use in an embodiment of the invention;
Figures 8A is a cross-sectional side view of an electrical ablation element for use in an embodiment of the invention;
Figure 8B is partial, blown-up, side view of the element of Fig. 8A; and
Figure 9 is a view of an electrical ablation element for use in an embodiment of the invention.

### Description of the Preferred Embodiments

There are described below and shown in the accompanying schematic diagrams various embodiments of an ablation system, or apparatus, in some embodiments an RF ablation system, for use in occluding a body vessel or for carrying out a medical treatment within the body of a patient. The preferred embodiment achieves this by causing coagulation of blood in order to close off the vessel. The apparatus may equally be used to effect ablation by means of heating the vessel wall, which can cause collapse of the vessel and fusing of the vessel wall tissue into a closed condition. Other embodiments can generate ablation heat deep into tissue, in order for instance to destroy a tumor or other diseased tissue or organ material.

The apparatus in its preferred embodiments can provide a very small diameter ablation element suitable for very small diameter vessels. The skilled person will appreciate that the teachings herein can be scaled up for use in larger sized vessels.

The drawings are schematic only and the various elements are not necessarily shown to scale. Not all of the components typically associated with such apparatus are shown, for the sake of clarity.

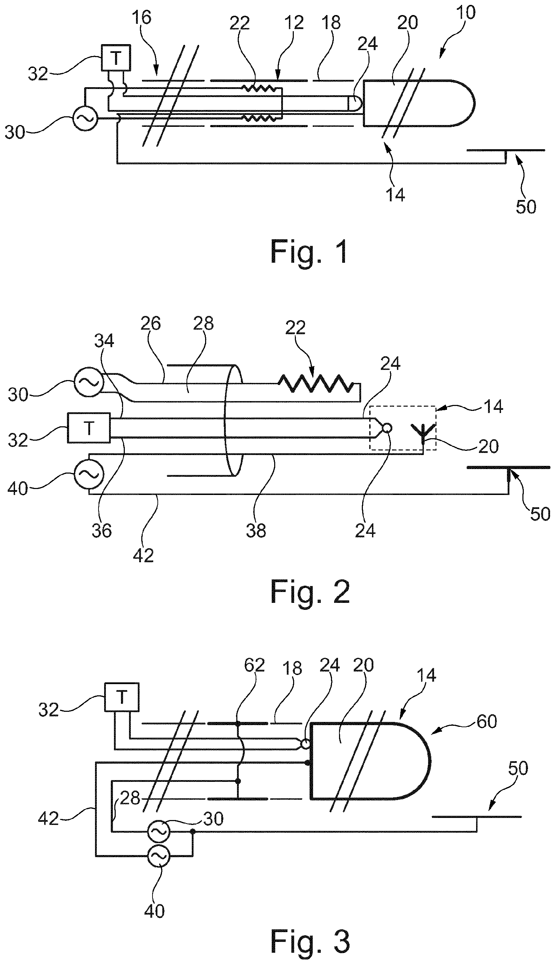

Referring first to Figure 1, this shows the principal components of a first embodiment of dual temperature ablation system 10. It is to be understood that the components are shown only in very schematic form and are not representative of how they will be manufactured in practice, which the skilled person will readily understand from common general knowledge.

The system 10 includes an elongate element 12 having a distal end 14 and a proximal end 16, and which may typically range from a few tens of centimetres to over one metre in length. The element 12 includes a catheter or cannula 18 having an internal lumen for housing the components of the apparatus. The catheter 18 is preferably made of a flexible material, such as polyurethane, silicon, latex, nylon, PTFE and the like. The catheter 18 may be substantially rigid, but is preferably flexible so as to be able to be passed through a patient's vasculature from a remote percutaneous entry point to a treatment site. It may have, for this purpose, internal strengthening elements such as braiding or a coil, to improve its resistance to kinking and to improve its pushability and torqueability. The catheter 18 is electrically non-conductive at least at its outermost surface.

The electrical components of the elongate element 12 are housed in the lumen of the catheter 12 and in this embodiment include an electrical ablation element in the form of a distal electrical ablation terminal 20, a heating device 22 and a temperature probe 24. The distal ablation terminal 20 is incorporated in or forms at least a part of the distal end 14 of the elongate element 12. In one embodiment, the distal ablation terminal 20 is a wire, in other embodiments the distal ablation terminal 20 is a conductive element attached to a dilator tip. The heating device may be disposed so as to reside wholly within the catheter 18 but in other embodiments may be at least partially exposed at the outer surface of the catheter 18.

The heating device 22 is, in this embodiment, longitudinally spaced from the ablation terminal 20, for purposes described below.

The temperature sensor probe 24 is preferably disposed between the heating device 22 and the ablation terminal 20, at a location in which it is able to measure temperature at the distal end 14 of the elongate element 12 and specifically the temperatures generated by the heating device 22 and by the ablation terminal 20.

With reference also to Figure 2, this shows schematically the arrangement of the components within the catheter 18. The heating device 22 is, in this embodiment, a resistive heating element which includes first and second electrical conductors 26, 28 coupled in this instance to an alternating current (AC) source 30 for feeding current through the resistive heating device 22. The current source 30 is controlled by a control unit described in detail below. The alternating current source may be of a high frequency, such as 100 kHz or above, as to avoid muscle or nerve stimulation.

In another embodiment the heating device 22 is a resistive element electrically insulated, for example by means of a polymer coating such as PTFE, coupled to a direct current source (DC). The use of direct current may advantageously minimize the electrical interference induced in sensitive electrical elements such as temperature probes.

The temperature probe 24 is coupled to a temperature sensor unit 32 through its own conductors 34, 36.

The distal electrical ablation terminal 20 is coupled by a conductor 38 to an AC source 40. In this embodiment, the source 40 is separate from that for the resistive heating element 22, although in other embodiments a common current source could be used. The system 10 also includes a second electrical terminal 50, in the form of a large surface area conductive pad which is coupled by a conductor 42 to the AC source 40. The conductive pad 50 forms, in this embodiment, the cathode of the ablation circuit constituted by the current source 40, the ablation terminal 20 (the latter being the anode) and the pad 50. As described below, the circuit is closed by conduction through the patient's body. The system shown in Figures 1 and 2, therefore, is a monopolar system in which only a single exposed conductive terminal, the terminal 20, is disposed endoluminally within the patient and the other conductive terminal, the pad 50, is kept outside the patient.

The system shown in Figures 1 and 2 could equally be constructed as a bipolar system, by having a second ablation terminal integral with the elongate element 12 and spaced from the distal electrical ablation terminal 20, in a manner which will be apparent to the skilled person. In such an embodiment, the heating device 22 will be disposed proximally of both of the ablation terminals.

As described in further detail below, the heating device 22 is used to generate heat at a first temperature, below ablation temperature, whereas the distal ablation terminal 20 is used to generate heat at a second, higher temperature to cause ablation. The system 10 is designed to effect a pre-heating step prior to ablation. The specific details are described below in connection with the control unit and operational method.

Referring now to Figure 3, this shows another embodiment of system 60 which has similar characteristics to the system 10 shown in Figures 1 and 2. The system 60 includes a distal electrical ablation terminal 20 disposed in or constituting the distal end 14 of the elongate element 12, a catheter or cannula 18 and a temperature sensor probe 24. The ablation system is again a monopolar system, having a cathode pad 50 which is disposed outside the patient for the procedure. The difference lies in the structure of a heating device, which in this embodiment is an electrical terminal 62 exposed at the outer surface of the sheath 18, rather than being a resistive element 32 as in the embodiment of Figures 1 and 2. The heating device 62 could be a large surface area device, such as a sleeve or the like, which generates gentle heating when energy is passed through it, in order to heat to the first, lower temperature.

The system 60 includes first and second AC current sources 30, 40, as with the embodiment of Figures 1 and 2, coupled respectively to the heating device 62 and the distal electrical ablation terminal 20.

In both embodiments, the distal ablation terminal 20 can simply be a wire which is electrically exposed as its distal end. When the wire forms the distal portion 14 of the system 10, 60, it is possible to construct a device having a very small transverse diameter, or footprint, suitable for treatment in or of narrow diameter vessels, for instance for neurological applications. The structure can also be scaled up for larger vessels or treatment of larger volumes, in which case the distal end 14 may include a nonconductive support or carrier to which the distal electrical ablation terminal is fitted. In such embodiments, the distal end 14 could form a part of a dilator tip, with the ablation terminal 20 disposed along the tip 14.

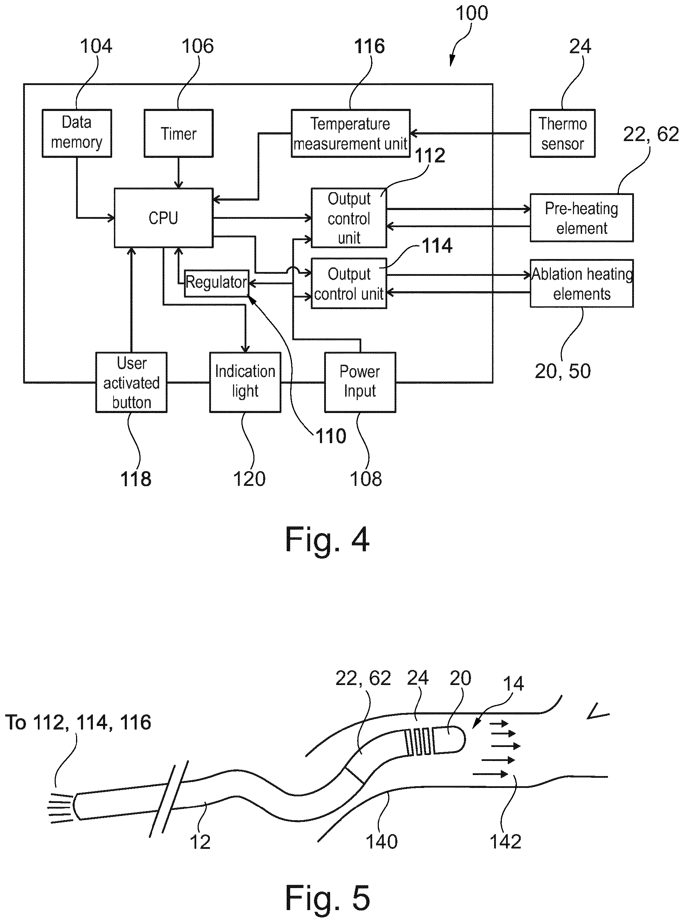

Referring now to Figure 4, this shows in schematic form the principal components of the preferred embodiment of control unit 100 for the system 10, 60 disclosed herein.

The control unit 100 includes a central processor 102, of suitable form, a data memory 104, a clock or timer 106, a power supply 108 and, as appropriate, a power regulator 110 for powering the central processor 102. Unit 100 also includes a pre-heating output control unit 112, coupled to the central processor 102, and an ablation output control unit 114, likewise coupled to the central processor 102. Both output control units 112, 114 are also coupled to the power supply 108 for receiving the necessary power. The pre-heating output control unit 112 is coupled to the pre-heating element 22, 62, whereas the ablation output control unit 114 is coupled to the ablation heating elements 20 and 50.

The output control units 112, 114 comprise the current sources 30, 40, respectively, for powering the pre-heating element 22/62 and the ablation electrodes 20 and 50, as well as circuitry for controlling the operation of the current sources, in a manner which will be apparent from the description which follows.

The control unit 100 also includes a temperature measurement unit 116 which is coupled to the temperature probe 24 and also to the central processor 102, in order to provide the central processor 102 with a measure of the temperature at the distal end of the elongate element 10 and as a result of the pre-heating and ablation temperatures.

The unit 110 also includes user input device 118, which in its simplest form could be a user activated button to operate the system 100. The unit 100 also typically includes an indicator light 120, which constitutes the or a part of an output interface and which in the preferred embodiment provides at least three distinct output states indicative of the system being: a) system active, b) successful operation and c) erroneous operation. The user input device could be more complex if desired, for instance to enable the user to set the heating and ablation temperatures, process times, and so on.

The temperature measurement unit 116 processes temperature information from the probe 24 and provides a temperature measurement to the central processor 102. The timer 106 provides a clock signal to the central processor 102, as is conventional in the art. The data memory 106 provides a data storage for storing variables, such as set points and the like, for use by the central processor 102. The regulator 110 regulates the power input to the central processor to appropriate levels, as is well known in the art.

The two output control units 112, 114 serve as controllable switches which route electrical power with adjustable intensity to the pre-heating and ablation heating elements, respectively. Each output control unit 112, 114 may be activated and have its output intensity adjusted by the central processor 102. In one embodiment, each output control unit 112, 114 comprises a simple electrical switch, such as a transistor, with changes in intensity being achieved by means of duty cycling the output by frequency modulation. In other embodiments output intensity can be adjusted by amplitude modulation.

The skilled person will appreciate that the control unit 100 may be provided with other features or functionality. In some embodiments, for example, the unit 100 may be provided with galvanic isolation of the outputs with respect to the inputs, redundancy of components to take into account possible component failure, monitoring elements for controlling the outputs of the output control units 112, 114 and so on.

Referring now to Figure 5, this shows in schematic form an embodiment of the elongate element 12 disposed within a vessel 140 for the ablation of blood, in order to occlude the vessel 140. In this embodiment, the distal end 14 of the elongate element 12 faces downstream, that is in the direction of blood flow 142, such that the pre-heating element 22/62 is upstream of the ablation electrode 20. The advantage of deploying the apparatus in this manner is that blood is pre-heated prior to reaching the ablation element 20, with the result that less energy and less time are required to heat the blood to ablation temperatures. As described in further detail below, and according to the invention, the pre-heating element 22/62 preferably pre-heats the blood to a temperature above body temperature, typically between 40-60 degrees and most preferably at around 50°C. The ablation element 20, on the other hand, will heat the blood to a significantly higher temperature, above 60°C and most preferably around 70°C.

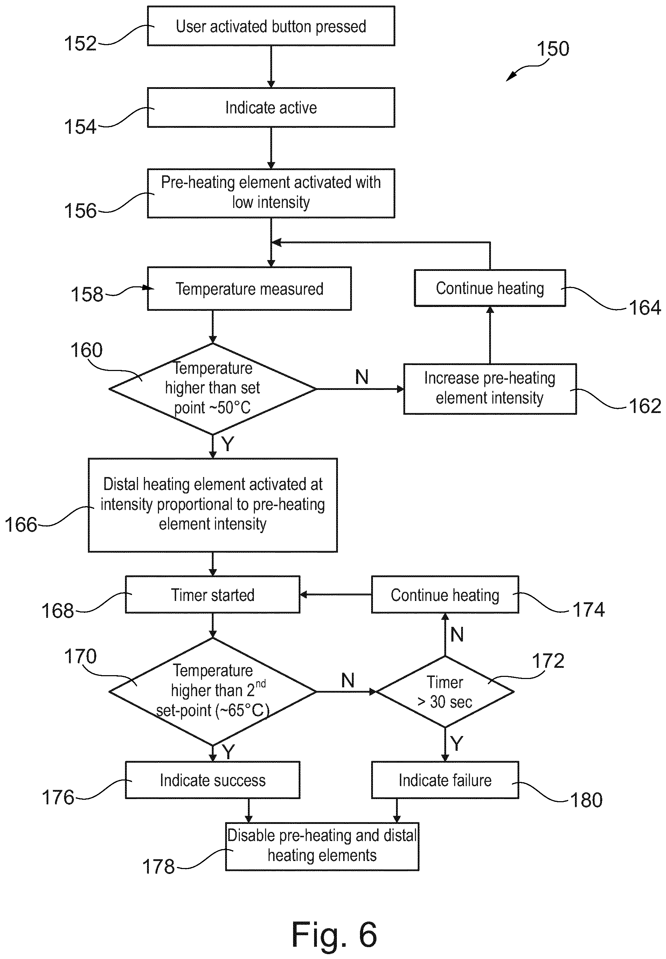

Referring now to Figure 6, this shows in flow diagram format the preferred method of operation of the system 10/60/100.

At step 152 the user activates the button 118, or other control input, to start the process. At step 154 the indicator light 120 is activated. At step 156 the central processor 102 activates the output control unit 112 in order to activate the pre-heating element 22, 62 to pre-heat blood or tissue or organ material at a first, low intensity. At step 158 temperature is measured by the temperature probe 24. If it is determined, at step 160, that the temperature is not higher than a predetermined set point, for example 50°C, the process passes to step 162 in which the intensity of energy supplied to the pre-heating element 22, 62 (typically current) is increased such that heating continues, at step 164. Steps 158-164 are repeated in cyclical manner until it is determined, at step 160, that the measured temperature has reached or exceeded the set point. When this occurs the process passes to step 166, when the ablation elements 20, 50 are activated, by activating the ablation output control unit 114 to generate an ablation current, at a higher intensity. The ablation intensity may initially be proportional to the intensity used in the pre-heating stage. At step 168, a timer is initiated and the process continues to step 170, at which temperature is measured and it is determined whether the temperature has risen to exceed a second set point, which may for example be between 60 to 70°C, in this example around 65°C.

If the second temperature threshold has not been reached, the process passes to step 172, at which it is determined if the ablation time has exceeded a certain period, in this example 30 seconds. If the predetermined time period has not been exceeded, the process passes to step 174, at which the ablation control unit continues to supply energy to the ablation elements 20, 50. The process continues through steps 168 to 174 in cyclical manner until, at step 170, it is determined that the measured temperature reaches or exceeds the second threshold. When this occurs, the process passes to step 176, at which the central processor 102 activates the indicator light 120 to indicate a successful operation, before passing to step 178 at which the pre-heating and ablation heating elements, that is the output control units 112, 114, are disabled. On the other hand, if it is determined at step 172 that the time period has exceeded the predetermined period, in this example 30 seconds, the process passes to step 180, at which the central processor 102 activates the indicator light 120 to indicate failure of the operation. It then passes to step 178, where the central processor 102 disables the output control units 112, 114 in order to disable the pre-heating and ablation heating elements.

The apparatus, system and process described above can provide small diameter electrode systems able to be inserted into very small vessels, whereas prior art devices contemplate much larger structures which are not suitable for small vessel ablation. The system and method disclosed herein are suitable for very small vessels of less than 3mm in diameter but can also be used in larger sized vessels.

The preferred embodiments can provide an improved ablation process with respect to consistency, reliability and safety, by providing in better controlled field with a higher proportion of energy delivered at the desired embolization area compared with surrounding tissue. As a result, collateral damage to tissue or organs can be minimised. In the preferred embodiment, the pre-heating element will heat blood or other material to be ablated to a first temperature threshold of, for example, 50°C and the system is preferably arranged to maintain that constant temperature of 50°C during the ablation process. In other words, once the first threshold temperature has been reached, it is maintained by the pre-heating device, and the ablation element is activated until the second temperature threshold (typically of up to 70°C) is reached. The ablation power is adjusted to maintain the secondary temperature for at most a pre-determined period of time, which may be around 30 seconds.

In the case where blood is pre-heated to, for example, approximately 50°C before the zone at which embolization is to be performed, less energy is required to bring the blood above its coagulation temperature at the actual embolization zone. As a result, a more focused embolization can take place, reducing the risk of overheating blood as ablation can be achieved at lower power levels.

A pre-heating temperature of around 50°C is preferred as this is comfortably below the 60°C barrier at which blood will coagulate, and it requires only a relatively small rise in temperature to cause ablation. In some implementations, the pre-heating temperature may be set to be greater than 50°C. It is preferred that the control unit 100 provides a given amount of pre-heating energy. In an embodiment, the system 100 uses a measure of blood flow (from a blood flow monitor) in order to determine a set amount of power to be supplied to the pre-heating element 22, 62 and then to apply ablation heating at a set power level for a set period of time. Another embodiment, as mentioned above, uses temperature feedback to ensure that the pre-heating temperature does not exceed a first threshold (for example 50°C) for a given time period. The skilled person will be able readily to appreciate the minor modifications to the control system and process to incorporate such functionalities.

The preferred embodiments use RF energy for ablation and may also use RF energy for the pre-heating element. The teachings herein, however, are not limited to an RF system for either of the heating devices.

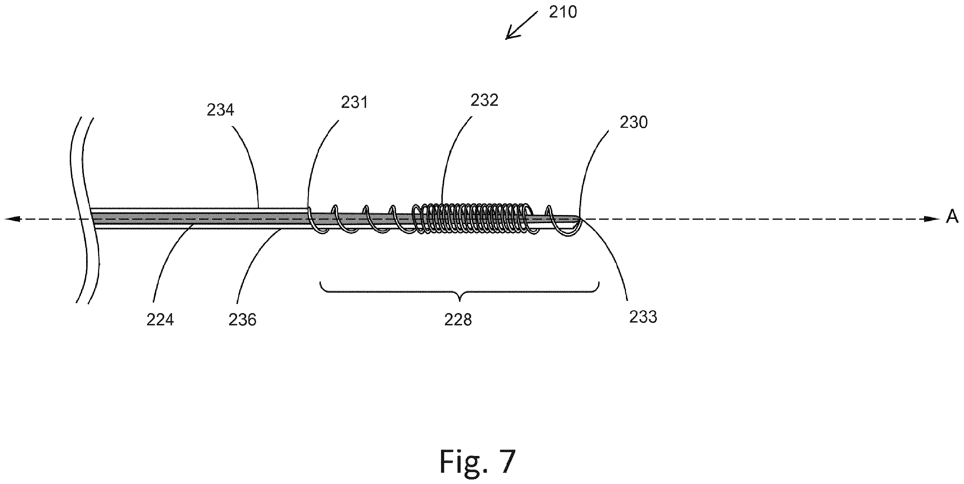

Fig. 7 depicts an electrical ablation element 210 in the form of a resistive heating device that can be used in any of the systems described above instead of, or in addition to, the electrical ablation terminals. The system is not depicted in full in Fig. 7 as many of the features are similar to those of the embodiments described above except where described below.

An assembly which uses the ablation element of Fig. 7 is preferably provided in the form of an elongate element in which the electrical components are housed in the lumen of a catheter, as for the embodiments described above, with the electrical ablation element being incorporated in or forming at least a part of a distal end of the elongate element.

In an embodiment using the electrical ablation element of Fig. 7, the pre-heating device can be arranged as described above. The pre-heating device is preferably longitudinally spaced in a proximal direction from the electrical ablation element so that it can for example be located upstream with respect to blood flow from the electrical ablation element when deployed in a blood vessel. This can allow blood to be preheated upstream of the electrical ablation element on its way to the ablation element so that the blood arriving at the ablation element is preheated and less time and energy is required to heat the blood to ablation temperatures.

The temperature sensor probe can be arranged as described above, that is between the electrical ablation element and the pre-heating device, at a location in which it is able to measure temperature at the distal end of the elongate element and specifically the temperatures generated by the pre-heating device and by the ablation element.

Continuing to refer to Fig. 7, the electrical ablation element 210 may have an elongate support 224, or mandrel, that extends from a proximal end to a distal end 230. The proximal and distal ends of the support 224 may correspond, respectively, to the proximal and distal ends of the elongate element. The support 224 may define a longitudinal axis A.

The electrical ablation element 210 may include a coil 232 disposed about the support 224, and the coil 232 may be electrically conductive. The coil 232 may have a first end 231 being disposed between the proximal and distal ends of the support 224, and the coil 232 may extend to a second end 233 being disposed at the distal end 230 of the support. The support 224 may have a distal segment 228 that supports the coil 232, or about which the coil 232 is disposed.

The coil may advantageously make good contact with a vessel wall during use.

The distal segment 228 may have an outer diameter that distally decreases to form a distal taper (as shown in Fig. 7). In another embodiment, the support 224, including its distal segment 228, may have an outer diameter being uniform from the proximal end to the distal end 230. The distal taper or tapered tip may provide the advantage of making the distal end 230 easier to track within the body vessel. Such distal taper may provide flexibility to track the whole ablation assembly. Additionally, the support 224 may have a curvature at the distal taper region or tip to facilitate tracking through the vasculature, such as may be seen in common guidewires.

The electrical ablation element 210 may further have a first wire 234 and a second wire 236. The first wire 234 may be electrically coupled or connected to a power or current source, for example by being coupled to the ablation output control unit 114 of a control unit such as described above. The first wire 234 may extend from the power or current source and along the longitudinal axis A to the first end 231 of the coil 232. The first wire 234 may be attached to the first end 231 of the coil 232 such that the connection provides an electrical coupling between the first wire 234 and the coil 232.

The second wire 236 may also be electrically coupled or connected to the power or current source, for example by being coupled to the ablation output control unit 114 of the control unit, and extend from the power or current source, along the longitudinal axis A, to the second end 233 of the coil 232. The second wire 236 may be attached to the second end 233 of the coil 232 such that the connection provides an electrical coupling between the second wire 236 and the coil 232. In this way, the electrical ablation element 210 may form a first circuit, or closed loop, along the path created by the power or current source, the first wire 234, the coil 232, and the second wire 236.

Although the first and second wires are described as being attached to the first and second ends of the coil, they can in other embodiments be attached at different locations of the coil provided that they can form a circuit including at least a resistive part of the coil.

In embodiments which use an electrical ablation element in the form of a resistive heating device, such as shown in Fig. 7, the ablation output control unit 114 of the control unit 100 can include the power or current source for powering the electrical ablation element as well as circuitry for controlling the operation of the power or current source in a manner as described herein. The current source for powering the electrical ablation element may be an alternating current source, which may be of high frequency, such as 100 kHz or above, so as to avoid muscle or nerve stimulation. In other embodiments, the current source for powering the electrical ablation element may be a direct current source.

A resistive part of the coil 232 has a higher electrical resistance than the other parts of the electrically conductive ablation element (e.g. 50-200 ohms). In this embodiment, the resistive part is the operative part of the electrical ablation element. The resistive part is configured so that the application of power to the wires (234, 236) causes current to flow through the resistive part, which can cause heating of the resistive part to the second temperature described above. The current may be transmitted through a circuit, including the control unit, the first wire 234, the coil 232, and the second wire 236. As the current flows through the higher resistance part of the coil 232, the coil heats up. This, in turn, causes ablation for example by causing embolization and/or ablation of blood surrounding the resistive part, and/or heating and consequential contraction of the vessel in the vicinity of the resistive part through the thermal energy at the resistive part. Structurally, the resistive part of the coil could be any part of the coil. In one example, the resistive part is the second end 233.

The step of transmitting a current through the coil may comprise contacting the vessel wall with the coil. Because the coil itself is caused to rise in temperature, direct contact with the vessel wall may be advantageous.

An assembly including the electrical ablation element of Fig.7 is preferably operated in accordance with the method of Fig. 6 but in which the electrical ablation element 210 is operated instead of the ablation elements 20, 50.

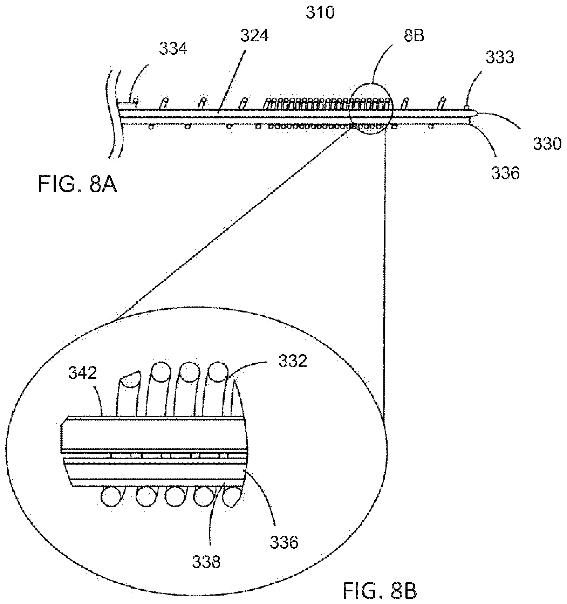

Figs. 8A-B depict details of an electrical ablation element similar to that of Fig. 7. The features of Figs. 8A and 8B can equally be used for the element of Fig.7. Fig. 8A shows a cross-sectional view of the electrical ablation element 310. Fig. 8B shows a blown-up view of the element around circle 8B. In Fig. 8A, the support 324 has a uniform outer diameter from the proximal end to the distal end 330. Additionally, either or both of the first and second wires may have an insulator disposed about their outer surface to electrically insulate or isolate them from the rest of the assembly. Insulator 338 is disposed about the second wire 336 in Fig. 8B. The coil 332 may be electrically insulated or isolated from the rest of the assembly, for example by having an insulator, such as a PTFE polymer coating, disposed about its outer surface.

Additionally, the electrical ablation element may have a shrink tubing. For example, the shrink tubing may extend around the support 324, the first wire 334, the second wire 336, and/or any other portion of the electrical ablation element. As one advantage, the shrink tubing may immobilize or bind the support 324, the first wire 334, and the second wire 336 in place so that they are immobilized relative to each other. The shrink tubing may also immobilize and/or extend over a part of the coil 332 to keep the coil 332 in position as the assembly is in use. Alternatively or additionally in Fig. 8B, the shrink tubing 342 may only be disposed about the support 324. In this configuration, the shrink tubing 342 may act to isolate or insulate the support 324 from the rest of the assembly.

The first wire 334 may be attached via a first lead and/or conductive wire to the control unit. Additionally, the second wire 336 may be attached to the control unit through a second lead and/or conductive wire. Various conductive wires and connectors may be used to electrically couple parts of the ablation element.

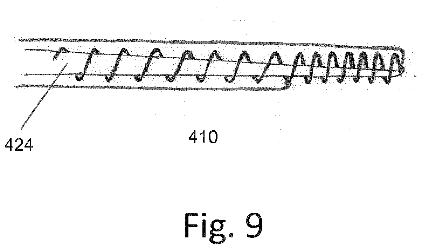

Figure 9 shows a view of a distal end of another ablation element 410 in many ways similar to that of Fig. 7 and Fig. 8A and 8B but in which the density of coil turns increases from the proximal end to the distal end of the support 424.

In some embodiments, the coil 232 of Fig. 7 can be used as an RF ablation element for performing RF ablation in the manner described above. In such embodiments, the coil 232 itself may have at least some part being electrically exposed. In such embodiments, the coil 232 may serve as the distal electrical ablation terminal, coupled to an AC source, such as in the ablation output control unit 114, by the first and/or second wires 234, 236. The second ablation terminal can be provided and coupled to the AC source as described above. For example, in a monopolar system, the conductive pad 50 shown in Figures 1 to 3 can be used as the second ablation terminal, with a circuit being formed by the control unit, the first wire 234, the coil 232, the pad 50, and the conductor 42. In a bipolar system, a return electrode may be disposed within the second wire 236. In this way, a circuit can be formed from the control unit, the first wire 234, the coil 232, and the return electrode being part of the second wire 236.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

## Claims

1. An ablation system for effecting ablation in a body vessel including:
an elongate ablation assembly having a proximal end and a distal end, and provided at or adjacent its distal end with a heating device (22, 62) and an electrical ablation element (20, 50, 210, 310, 410); and
a control unit connected to the heating device (22, 62) and the electrical ablation element (20, 50, 210, 310, 410), the control unit being configured to actuate the heating device (22, 62) to provide heating to blood to a first temperature upstream of the electrical ablation element in a first mode and to actuate the electrical ablation element (20, 50, 210, 310, 410) to provide heating to a second temperature in a second mode, the first temperature being below an ablation temperature and the second temperature being at or above an ablation temperature, the first temperature being between 40°C and 60°C.

2. An ablation system according to claim 1, wherein the ablation assembly is for deployment in and effecting ablation in a blood vessel and wherein the heating device (22, 62) is longitudinally spaced from the electrical ablation element (20, 50, 210, 310, 410).

3. An ablation system according to claim 1 or 2, wherein the heating device (22, 62) is longitudinally spaced from the electrical ablation element (20, 50, 210, 310, 410) so as to provide longitudinally spaced heating.

4. An ablation system according to any preceding claim, wherein the electrical ablation element(20, 50, 210, 310, 410) includes a resistive heating device.

5. An ablation system according to any preceding claim, wherein the electrical ablation element (20, 50, 210, 310, 410) includes at least one electrical ablation terminal.

6. An ablation system according to any preceding claim, wherein the control unit is configured to actuate the heating device (22, 62) to heat to a temperature below around 60 degrees centigrade and to actuate the electrical ablation element (20, 50, 210, 310, 410) to a temperature above about 60 degrees centigrade.

7. An ablation system according to any preceding claim, wherein the control unit is configured to actuate the heating device (22, 62) to heat to a temperature of around 50 degrees centigrade.

8. An ablation system according to any preceding claim, wherein the control unit is operable to actuate the heating device (22, 62) before the electrical ablation element.

9. An ablation system according to any preceding claim, wherein the heating device (22, 62) is a resistive heating device.

10. An ablation system according to any preceding claim, wherein the heating device (22, 62) is a closed loop heating element.

11. An ablation system according to any preceding claim, wherein the elongate ablation assembly includes a distal end and a proximal end, the electrical ablation element being disposed at the distal end of the device, the heating device being disposed at or proximate the distal end.

12. An ablation system according to any preceding claim, wherein the elongate ablation assembly includes a temperature sensor disposed to measure temperature at least at the heating device.

## Patentansprüche

1. Ablationssystem zur Durchführung von Ablation in einem Körpergefäß, umfassend:
eine langgestreckte Ablationsbaugruppe mit einem proximalen Ende und einem distalen Ende, die an oder nahe ihrem distalen Ende mit einer Heizvorrichtung (22, 62) und einem elektrischen Ablationselement (20, 50, 210, 310, 410) versehen ist; und
eine Steuereinheit, die mit der Heizvorrichtung (22, 62) und dem elektrischen Ablationselement (20, 50, 210, 310, 410) verbunden ist, wobei die Steuereinheit dafür gestaltet ist, in einem ersten Modus die Heizvorrichtung (22, 62) anzusteuern, um Erwärmen von Blut stromaufwärts bezogen auf das elektrische Ablationselement auf eine erste Temperatur zu bewirken, und in einem zweiten Modus das elektrische Ablationselement (20, 50, 210, 310, 410) anzusteuern, um Erwärmen auf eine zweite Temperatur zu bewirken, wobei die erste Temperatur unter einer Ablationstemperatur liegt und die zweite Temperatur über einer Ablationstemperatur liegt, wobei die erste Temperatur zwischen 40 °C und 60 °C beträgt.

2. Ablationssystem gemäß Anspruch 1, wobei die Ablationsbaugruppe zum Einführen in ein und Durchführen von Ablation in einem Blutgefäß dient und wobei die Heizvorrichtung (22, 62) in Längsrichtung von dem elektrischen Ablationselement (20, 50, 210, 310, 410) beabstandet ist.

3. Ablationssystem gemäß Anspruch 1 oder 2, wobei die Heizvorrichtung (22, 62) in Längsrichtung von dem elektrischen Ablationselement (20, 50, 210, 310, 410) beabstandet ist, um in Längsrichtung beabstandetes Heizen bereitzustellen.

4. Ablationssystem gemäß einem der vorstehenden Ansprüche, wobei das elektrische Ablationselement (20, 50, 210, 310, 410) eine Widerstandsheizvorrichtung aufweist.

5. Ablationssystem gemäß einem der vorstehenden Ansprüche, wobei das elektrische Ablationselement (20, 50, 210, 310, 410) wenigstens eine elektrische Ablations-Endvorrichtung enthält.

6. Ablationssystem gemäß einem der vorstehenden Ansprüche, wobei die Steuereinheit dafür gestaltet ist, die Heizvorrichtung (22, 62) anzusteuern, auf eine Temperatur von unter etwa 60 Grad Celsius zu heizen, und das elektrische Ablationselement (20, 50, 210, 310, 410) auf eine Temperatur über etwa 60 Grad Celsius anzusteuern.

7. Ablationssystem gemäß einem der vorstehenden Ansprüche, wobei die Steuereinheit dafür gestaltet ist, die Heizvorrichtung (22, 62) anzusteuern, auf eine Temperatur von etwa 50 Grad Celsius zu heizen.

8. Ablationssystem gemäß einem der vorstehenden Ansprüche, wobei die Steuereinheit funktionsfähig ist, die Heizvorrichtung (22, 62) vor dem elektrischen Ablationselement anzusteuern.

9. Ablationssystem gemäß einem der vorstehenden Ansprüche, wobei die Heizvorrichtung (22, 62) eine Widerstandsheizvorrichtung ist.

10. Ablationssystem gemäß einem der vorstehenden Ansprüche, wobei die Heizvorrichtung (22, 62) ein Heizelement mit geschlossener Schleife ist.

11. Ablationssystem gemäß einem der vorstehenden Ansprüche, wobei die langgestreckte Ablationsbaugruppe ein distales Ende und ein proximales Ende aufweist, wobei das elektrische Ablationselement an dem distalen Ende der Vorrichtung angeordnet ist, wobei die Heizvorrichtung an oder nahe dem distalen Ende angeordnet ist.

12. Ablationssystem gemäß einem der vorstehenden Ansprüche, wobei die langgestreckte Ablationsbaugruppe einen Temperatursensor aufweist, der angeordnet ist, um wenigstens an der Heizvorrichtung Temperatur zu messen.

## Revendications

1. Système d'ablation permettant de réaliser une ablation dans un vaisseau sanguin, comprenant :
un ensemble allongé d'ablation possédant une extrémité proximale et une extrémité distale, et doté d'un dispositif chauffant (22, 62) au niveau de son extrémité distale ou de manière adjacente à celle-ci et d'un élément électrique d'ablation (20, 50, 210, 310, 410) ; et
une unité de commande connectée au dispositif chauffant (22, 62) et à l'élément électrique d'ablation (20, 50, 210, 310, 410), l'unité de commande étant configurée pour actionner le dispositif chauffant (22, 62) pour fournir au sang un chauffage à une première température en amont de l'élément électrique d'ablation dans un premier mode et pour actionner l'élément électrique d'ablation (20, 50, 210, 310, 410) pour fournir un chauffage à une seconde température dans un second mode, la première température étant inférieure à une température d'ablation et la seconde température étant égale ou supérieure à une température d'ablation, la première température étant entre 40 °C et 60 °C.

2. Système d'ablation selon la revendication 1, dans lequel l'ensemble d'ablation est destiné à un déploiement dans un vaisseau sanguin et à la réalisation d'une ablation dans celui-ci et dans lequel le dispositif chauffant (22, 62) est espacé longitudinalement de l'élément électrique d'ablation (20, 50, 210, 310, 410).

3. Système d'ablation selon la revendication 1 ou 2, dans lequel le dispositif chauffant (22, 62) est espacé longitudinalement de l'élément électrique d'ablation (20, 50, 210, 310, 410) de façon à fournir un chauffage espacé longitudinalement.

4. Système d'ablation selon l'une quelconque des revendications précédentes, dans lequel l'élément électrique d'ablation (20, 50, 210, 310, 410) comprend un dispositif chauffant résistif.

5. Système d'ablation selon l'une quelconque des revendications précédentes, dans lequel l'élément électrique d'ablation (20, 50, 210, 310, 410) comprend au moins une borne électrique d'ablation.

6. Système d'ablation selon l'une quelconque des revendications précédentes, dans lequel l'unité de commande est configurée pour actionner le dispositif chauffant (22, 62) pour qu'il chauffe à une température inférieure à environ 60 degrés Celsius et pour actionner l'élément électrique d'ablation (20, 50, 210, 310, 410) à une température supérieure à environ 60 degrés Celsius.

7. Système d'ablation selon l'une quelconque des revendications précédentes, dans lequel l'unité de commande est configurée pour actionner le dispositif chauffant (22, 62) pour qu'il chauffe à une température d'environ 50 degrés Celsius.

8. Système d'ablation selon l'une quelconque des revendications précédentes, dans lequel l'unité de commande est exploitable pour actionner le dispositif chauffant (22, 62) avant l'élément électrique d'ablation.

9. Système d'ablation selon l'une quelconque des revendications précédentes, dans lequel le dispositif chauffant (22, 62) est un dispositif chauffant résistif.

10. Système d'ablation selon l'une quelconque des revendications précédentes, dans lequel le dispositif chauffant (22, 62) comprend un élément chauffant à boucle fermée.

11. Système d'ablation selon l'une quelconque des revendications précédentes, dans lequel l'ensemble allongé d'ablation comprend une extrémité distale et une extrémité proximale, l'élément électrique d'ablation étant disposé au niveau de l'extrémité distale du dispositif, le dispositif chauffant étant disposé au niveau de l'extrémité distale ou à proximité de celle-ci.

12. Système d'ablation selon l'une quelconque des revendications précédentes, dans lequel l'ensemble allongé d'ablation comprend un capteur de température disposé pour mesurer une température au moins au niveau du dispositif chauffant.
